(19) Europäisches Patentamt
European Patent Office
Office européen des brevets

(11) EP 4 129 410 A1

(12) **EUROPEAN PATENT APPLICATION**
published in accordance with Art. 153(4) EPC

(43) Date of publication:
**08.02.2023 Bulletin 2023/06**

(21) Application number: **21781977.0**

(22) Date of filing: **22.03.2021**

(51) International Patent Classification (IPC):
***A61Q 1/00*** *(2006.01)* ***A61Q 1/02*** *(2006.01)*
***A61Q 1/12*** *(2006.01)* ***A61K 8/29*** *(2006.01)*
***A61K 8/73*** *(2006.01)* ***A61K 8/81*** *(2006.01)*
***A61K 8/89*** *(2006.01)* ***A61K 8/92*** *(2006.01)*

(52) Cooperative Patent Classification (CPC):
**A61K 8/29; A61K 8/31; A61K 8/73; A61K 8/81; A61K 8/89; A61K 8/92; A61Q 1/00; A61Q 1/02; A61Q 1/12**

(86) International application number:
**PCT/JP2021/011782**

(87) International publication number:
**WO 2021/200352 (07.10.2021 Gazette 2021/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **01.04.2020 JP 2020066246**

(71) Applicant: **Kao Corporation**
**Chuo-ku**
**Tokyo 103-8210 (JP)**

(72) Inventors:
- **IIDA, Masayuki**
  **Odawara-shi, Kanagawa 250-0002 (JP)**
- **KAWAMOTO, Sachiyo**
  **Odawara-shi, Kanagawa 250-0002 (JP)**
- **NAKAJIMA, Ryota**
  **Odawara-shi, Kanagawa 250-0002 (JP)**
- **MIYAZAKI, Yukihiro**
  **Odawara-shi, Kanagawa 250-0002 (JP)**

(74) Representative: **Hoffmann Eitle**
**Patent- und Rechtsanwälte PartmbB**
**Arabellastraße 30**
**81925 München (DE)**

# (54) MAKEUP APPLICATION METHOD

(57) Provided is a makeup application method by which no uncomfortable feeling specific to polymers is caused immediately after application, the film has high adhesiveness, and coverage can be achieved with the film appearing fine-textured in a natural state.

A makeup application method comprising the steps of:
(A) applying to the skin a composition X comprising components (A1) and (A2):
(A1) a polymer comprising a norbornane structure and/or a silicone-modified Pullulan; and
(A2) a volatile oil; and

(B) applying to the skin a composition Y comprising a component (B1), other than the composition X:
(B1) one or more selected from the group consisting of powders.

EP 4 129 410 A1

## Description

### Field of the Invention

The present invention relates to a makeup application method.

### Background of the Invention

Conventionally, cosmetics compounded with a film-forming polymer are known which are intended to improve the uniformity of a makeup film and to extend makeup lasting. For example, Patent Literature 1 describes that a cosmetic containing a film-forming polymer in which a predetermined functional group is introduced into the skeleton of a polycycloolefin polymer allows makeup to last and achieves a favorable feel.

### Patent Literature

Patent Literature 1: JP-A-2012-17317

### Summary of the Invention

### Technical Problem

However, cosmetics comprising a film-forming polymer have had such problems that an uncomfortable feeling is caused immediately after application and the film does not look nice.

### Solution to Problem

The present inventors found that no uncomfortable feeling specific to polymers is caused immediately after application, the film has high adhesiveness, and coverage can be achieved with the film appearing fine-textured in a natural state by applying a composition Y comprising one or more selected from the group consisting of powders before or after application of a composition X comprising a specific film-forming polymer and a volatile oil, and accomplished the present invention.

The present invention relates to a makeup application method comprising the steps of:

(A) applying to the skin a composition X comprising components (A1) and (A2):

(A1) a polymer comprising a norbornane structure and/or a silicone-modified Pullulan; and
(A2) a volatile oil; and

(B) applying to the skin a composition Y comprising a component (B1), other than the composition X:
(B1) one or more selected from the group consisting of powders.

The present invention also relates to a cosmetic kit to be applied to the skin, comprising:

(A) a composition X comprising components (A1) and (A2):

(A1) a polymer comprising a norbornane structure and/or a silicone-modified Pullulan; and
(A2) a volatile oil; and

(B) a composition Y comprising a component (B1), other than the composition X:
(B1) one or more selected from the group consisting of powders.

### Advantageous Effects of Invention

According to the present invention, no uncomfortable feeling specific to polymers is caused immediately after application, the film has high adhesiveness, and coverage can be achieved with the film appearing fine-textured in a natural state.

The present invention also has excellent effects of stretching skin wrinkles to make them less noticeable and of lifting the skin up to pull sagging of the skin up.

The term "skin wrinkles" means roughness or folds that occur on the skin surface, which are changes in the shape that occur because of sagging of the skin. Skin wrinkles are likely to occur around the mouth and the eyes, on the forehead, the neck, and the body, and the like. Small folds are fine wrinkles, and large folds are large wrinkles, which occur with a flow of enlarged skin pores of nasolabial folds, cheek folds, and the like.

Detailed Description of the Invention

<Component (A1)>

In the present invention, the component (A1) used in the composition X is (A11) a polymer comprising a norbornane structure and/or (A12) a silicone-modified Pullulan.

When the composition comprising the component (A1) is applied to the skin by coating or the like and then dried, shrinkage occurs in the film comprising the component (A1) as well as in the skin surface attached to the film. The component (A1) is also excellent in bend resistance, and thus a film comprising the component (A1) is unlikely to crack and likely to shrink while following the skin shape. Accordingly, also when the film comprising the component (A1) shrinks, sufficient adhesiveness between the film and the skin surface can be obtained.

Among the component (A1), in (A11)the polymer comprising a norbornane structure, the norbornane structure means a structure of the following formula. It is sufficient that the polymer (A11) has the structure of the following formula at an arbitrary site in the polymer (A11).

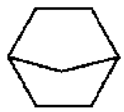

The component (A11) is not particularly limited as long as the component (A11) is a polymer comprising the norbornane structure described above in the molecule, but the component (A11) is even more preferably a norbornane structure-comprising silicone-modified polymer, in view of increasing shrinkage and adhesiveness to the skin of the coating film by means of the mechanism of action mentioned above.

Examples of norbornane structure-comprising silicone-modified polymers include polymers having a repeating unit of the following formula (1) or (2):

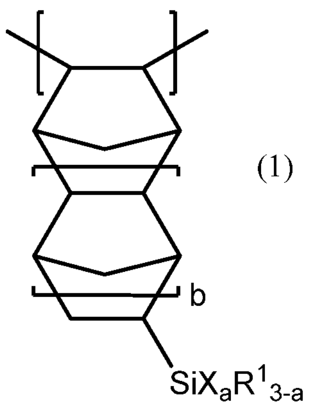

(1)

wherein $R^1$ is each independently an alkyl group having one or more and 12 or less carbon atoms, X is a group of the following formula (i). a is an integer of 1 or above and 3 or below, and b is an integer of 0 or above and 2 or below.

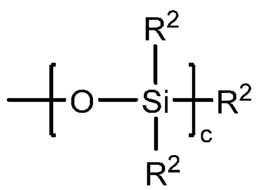

(i)

wherein $R^2$ is each independently a hydrocarbon group having one or more and 12 or less carbon atoms, and c is an integer of 1 or above and 5 or below.

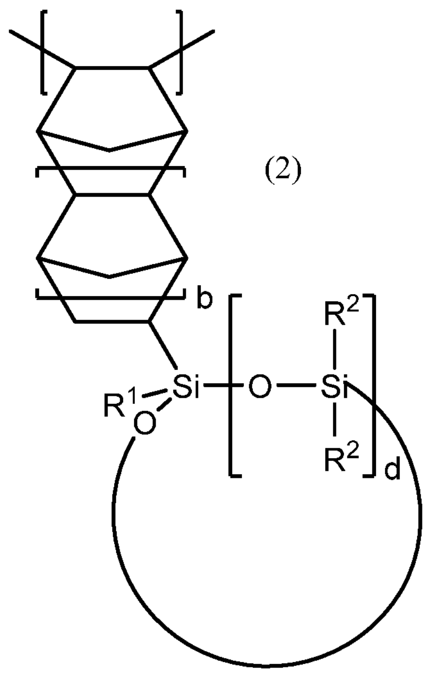

(2)

wherein $R^1$, $R^2$, and b are the same as described above, and d is an integer of 2 or above and 5 or below.

In the formula (1), $R^1$ is each independently an alkyl group having one or more and 12 or less carbon atoms, and in view of the skin wrinkle improving effect and versatility, $R^1$ is preferably methyl group, ethyl group, n-propyl group, butyl group, or pentyl group, more preferably methyl group.

X is a group of the formula (i). In the formula (i), $R^2$ is each independently a hydrocarbon group having one or more and 12 or less carbon atoms. In view of the skin wrinkle improving effect and in view of versatility, $R^2$ is preferably an alkyl group having one or more and 12 or less carbon atoms or an aryl group having six or more and 12 or less carbon atoms, more preferably an alkyl group or a phenyl group having one or more and 12 or less carbon atoms, even more preferably an alkyl group having one or more and three or less carbon atoms, even more preferably methyl group, c is an integer of 1 or above and 5 or below, and in view of versatility, c is preferably equal to 1. Specifically, X is preferably a trimethylsiloxy group.

a is an integer of 1 or above and 3 or below, and the polymer may be, for example, a polymer having a mixture of a repeating unit with a being 2 and a repeating unit with a being 3. In view of versatility, a is preferably 3. b is an integer of 0 or above and 2 or below, and in the same view, b is preferably 0, 1, or a combination thereof, more preferably 0.

In the formula (2), $R^1$, $R^2$, and b are the same as described above. d is an integer of 2 or above and 5 or below, and in view of versatility, d is preferably equal to 2. In the cyclic silicone structure in the formula (2), it is preferred in the same view that $R^1$ and $R^2$ are methyl group, and that d is 2 or 3. Specifically, the cyclic silicone structure in the formula (2) is preferably a structure of the following formula (4) or (5) :

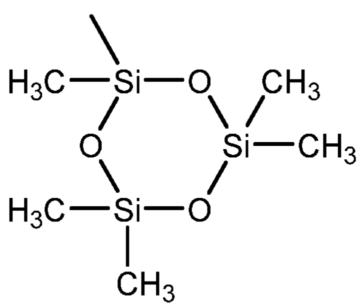

(4)

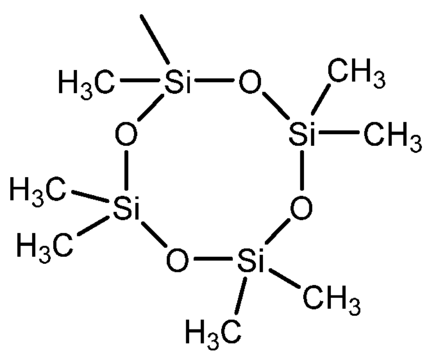

(5)

In view of the skin wrinkle improving effect, the proportion of repeating units of the formula (1) or (2) in a norbornane structure-comprising silicone-modified polymer is preferably 10% or higher, more preferably 30% or higher, even more preferably 50% or higher of the number of all repeating units in the polymer. The upper limit is 100%, and

the proportion is preferably 95% or lower, more preferably 90% or lower, even more preferably 70% or lower. In a norbornane structure-comprising silicone-modified polymer, the specific range of the number of repeating units of the formula (1) or (2) is preferably from 10% to 100%, more preferably from 10% to 95%, even more preferably from 30% to 90%, even more preferably from 50% to 70% of the number of all repeating units in the polymer.

A norbornane structure-comprising silicone-modified polymer may have a repeating unit of the following formula (3) in addition to the repeating unit of the formula (1) or (2).

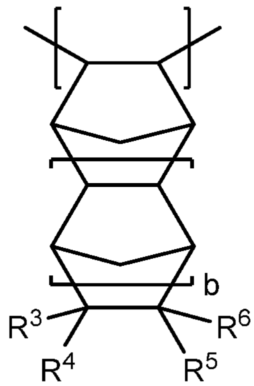
(3)

wherein $R^3$ to $R^6$ are each independently a substituent group selected from the group consisting of a hydrogen atom; a halogen atom; an alkyl group, an alkenyl group, a cycloalkyl group, an aryl group, an alkoxy group, an aryloxy group, and a halogenated hydrocarbon group which have one or more and 10 or less carbon atoms; an oxetanyl group, an alkoxycarbonyl group, a polyoxyalkylene group, a polyglyceryl group, and an alkoxysilyl group. Two groups chosen from $R^3$ to $R^6$ may be taken together to form an aliphatic ring structure, an aromatic ring structure, a carboimide group, or an acid anhydride group. b is the same as described above.

In view of the skin wrinkle improving effect, the proportion of the above-described repeating units of the formula (3) in a norbornane structure-comprising silicone-modified polymer is preferably 90% or lower, more preferably 70% or lower, even more preferably 50% or lower of the number of all repeating units in the polymer. Further, when the above-described repeating units of the formula (3) are contained, the proportion thereof is preferably 5% or higher, more preferably 10% or higher, even more preferably 30% or higher of the number of all repeating units in the polymer. In a norbornane structure-comprising silicone-modified polymer, the specific range of the number of repeating units of the formula (3) is preferably from 5% to 90%, more preferably from 10% to 70%, even more preferably from 30% to 50% of the number of all repeating units in the polymer.

The proportion of the above-described repeating units of the formulas (1) to (3) in a norbornane structure-comprising silicone-modified polymer can be obtained by $^1$H-NMR measurement.

The norbornane structure-comprising silicone-modified polymer is preferably a silicone-modified polynorbornene, more preferably a silicone-modified polynorbornene of the following formula (6):

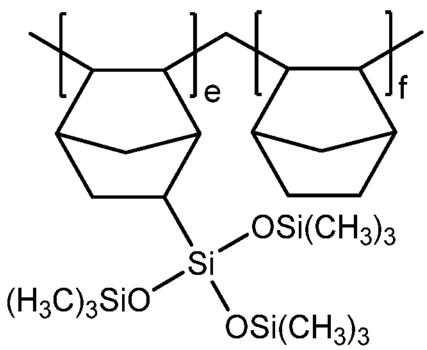
(6)

wherein e and f are the number of repeating units and are each independently an integer of 1 or above.

In view of the skin wrinkle improving effect, the ratio of e and f, e/f, in the formula (6) is preferably from 20/80 to 90/10 (mol/mol), more preferably from 30/70 to 80/20 (mol/mol), even more preferably from 50/50 to 70/30 (mol/mol).

In view of balancing shrinkage and bend resistance and in view of the skin wrinkle improving effect, the number average molecular weight (Mn) of a norbornane structure-comprising silicone-modified polymer is preferably 50,000 or

more, more preferably 100,000 or more, even more preferably 200,000 or more, and preferably 2,000,000 or less, more preferably 1,500,000 or less, even more preferably 800,000 or less, even more preferably 600,000 or less. The specific range of the number average molecular weight (Mn) of a norbornane structure-comprising silicone-modified polymer is preferably from 50,000 to 2,000,000, more preferably from 100,000 to 1,500,000, even more preferably from 200,000 to 800,000, even more preferably from 200,000 to 600,000.

The number average molecular weight (Mn) of the polymer can be measured by gel filtration chromatography (GPC) using polystyrene as a reference substance, and specifically by the methods described in the examples.

The component (A11) can be obtained by, for example, a known method of addition polymerization of a cyclic olefin monomer which comprises a norbornane structure or can form a norbornane structure.

For example, when the component (A11) is the above-described norbornane structure-comprising silicone-modified polymer having the repeating unit of the formula (1) or (2), it can be obtained by addition polymerization of a cyclic olefin monomer of the following formula (1a) or (2a). Further, a cyclic olefin monomer of the formula (3a) may be copolymerized.

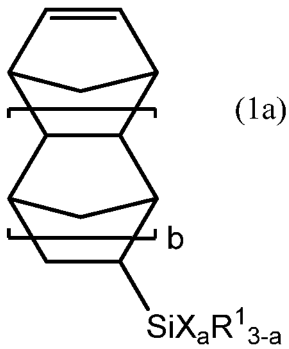

(1a)

wherein $R^1$ is each independently an alkyl group having one or more and 12 or less carbon atoms, and X is a group of the following formula (i). a is an integer of 1 or above and 3 or below, and b is an integer of 0 or above and 2 or below.

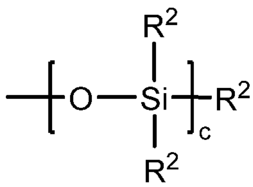

(i)

wherein $R^2$ is each independently a hydrocarbon group having one or more and 12 or less carbon atoms, and c is an integer of 1 or above and 5 or below.

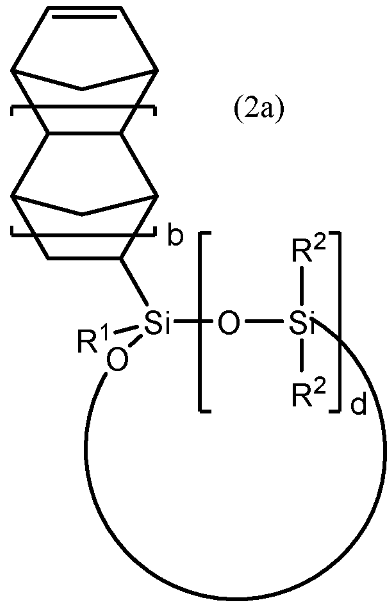

(2a)

wherein $R^1$, $R^2$, and b are the same as described above, and d is an integer of 2 or above and 5 or below.

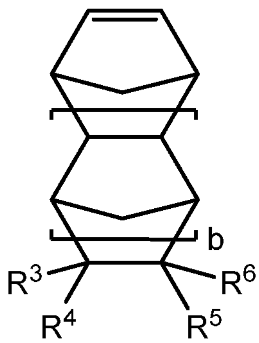

(3a)

wherein $R^3$ to $R^6$ are each independently a substituent group selected from the group consisting of a hydrogen atom; a halogen atom; an alkyl group, an alkenyl group, a cycloalkyl group, an aryl group, an alkoxy group, an aryloxy group, and a halogenated hydrocarbon group which have one or more and 10 or less carbon atoms; an oxetanyl group, an alkoxycarbonyl group, a polyoxyalkylene group, a polyglyceryl group, and an alkoxysilyl group. Two groups chosen from $R^3$ to $R^6$ may be taken together to form an aliphatic ring structure, an aromatic ring structure, a carboimide group, or an acid anhydride group. b is the same as described above.

When the above-described cyclic olefin monomer of the formula (3a) is copolymerized, the use amount thereof is, in view of the skin wrinkle improving effect, preferably 90 mol% or less, more preferably 70 mol% or less, even more preferably 50 mol% or less, and preferably 5 mol% or more, more preferably 10 mol% or more, even more preferably 30 mol% or more, assuming the amount of all monomers used for polymerization as 100 mol%.

The above-described silicone-modified polynorbornene of the formula (6) can be obtained by addition polymerization of tris(trimethylsiloxy)silylnorbornene of the following formula (6a) and norbornene:

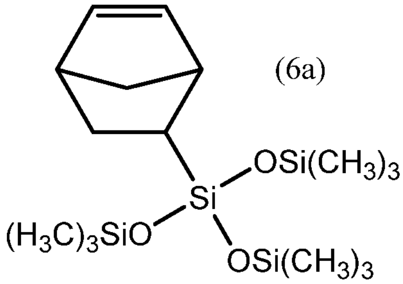

(6a)

In view of increasing shrinkage and adhesiveness to the skin of the coating film, the copolymerization ratio of tris(trimethylsiloxy)silylnorbornene and norbornene is preferably from 20/80 to 90/10 (mol/mol), more preferably from 30/70 to 80/20 (mol/mol), even more preferably from 50/50 to 70/30 (mol/mol).

Of note, all the repeating units of the formulas (1) to (3) and (6) represent a unit of a 2,3-addition structure of a cyclic olefin monomer as a raw material monomer and may contain a unit of a 2,7-addition structure obtained by addition polymerization of the cyclic olefin monomer.

The norbornane structure-comprising silicone-modified polymer is preferably a (norbornene/tris(trimethylsiloxy)silylnorbornene) copolymer, i.e., a compound which is expressed as NORBORNENE/TRIS(TRIMETHYLSILOXY)SILYLNORBORNENE COPOLYMER, as the INCI name (International Cosmetic Ingredient Dictionary and Handbook, 16th Edition, Volume 2, 2016, p.2274).

Examples of commercially available norbornane structure-comprising silicone-modified polymers include "NBN-30-ID" (an isododecane solution of a norbornene/tris(trimethylsiloxy)silylnorbornene) copolymer) manufactured by Shin-Etsu Chemical Co., Ltd.

Among the component (A1), examples of (A12) silicone-modified Pullulans include Pullulans having a silicone structure in a side chain. Specifically, in view of increasing shrinkage and adhesiveness to the skin of the coating film, a silicone-modified Pullulan in which at least some hydrogen atoms of an OH group in the Pullulan is substituted with a group of the following formula (7) is preferred:

$$-Z^1-SiX_aR^1_{3-a} \quad (7)$$

wherein $Z^1$ is a single bond or divalent organic group. $R^1$, X, and a are the same as described above, and in the same view, X is preferably trimethylsiloxy group, and a is preferably 3.

In the same view, in the formula (7), $Z^1$ is preferably a divalent organic group, more preferably a divalent group of the following formula (8) or (9), even more preferably a divalent group of the following formula (9) .

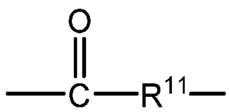

(8)

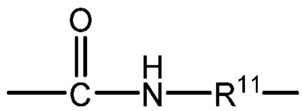

(9)

wherein $R^{11}$ is an alkylene group having one or more and 10 or less carbon atoms, and examples thereof include methylene group, ethylene group, trimethylene group, propylene group, and butylene group. Of these, in the same view, ethylene group, trimethylene group, and propylene group are preferred, and trimethylene group and propylene group are more preferred.

Examples of commercially available silicone-modified Pullulans include "TSPL-30-ID" (an isododecane solution of tri(trimethylsiloxy)silyl propyl carbamide acid Pullulan) and "TSPL-30-D5" (a cyclopentasiloxane solution of tri(trimethylsiloxy)silyl propyl carbamide acid Pullulan), which are manufactured by Shin-Etsu Chemical Co., Ltd.

In view of increasing shrinkage and adhesiveness to the skin of the coating film, the component (A1) is preferably (A11) the polymer comprising a norbornane structure, more preferably a norbornane structure-comprising silicone-modified polymer.

As the component (A1), one or more of polymers comprising a norbornane structure and silicone-modified Pullulans can be used in combination. In view of increasing shrinkage and adhesiveness to the skin of the coating film, the content of the solid content is preferably 0.01 mass% or more, more preferably 1 mass% or more, even more preferably 2 mass% or more, even more preferably 4 mass% or more, even more preferably 5 mass% or more, and preferably 30 mass% or less, more preferably 28 mass% or less, even more preferably 25 mass% or less, even more preferably 20 mass% or less, even more preferably 18 mass% or less in the composition X. Further, the content of the solid content of the component (A1) is preferably from 0.01 to 30 mass%, more preferably from 1 to 28 mass%, even more preferably from 2 to 25 mass%, even more preferably from 4 to 20 mass%, even more preferably from 5 to 18 mass% in the composition X.

<Component (A2)>

The component (A2) used in the present invention is a volatile oil. Volatility refers to having a flash point of

35°C or higher and less than 90°C.

As the volatile oil of the component (A2), one or more selected from the group consisting of volatile silicone oils and volatile hydrocarbon oils are preferable.

Examples of volatile silicone oils include straight-chain dimethylpolysiloxanes such as hexamethyldisiloxane, octamethyltrisiloxane, dimethylpolysiloxane (1cs), dimethylpolysiloxane (1.5cs), and dimethylpolysiloxane (2cs); branched-chain siloxanes such as methyl trimethicone, tris(trimethylsilyl)methylsilane, and tetrakis(trimethylsilyl)silane; and cyclic dimethylsiloxanes such as octamethylcyclotetrasiloxane, decamethylcyclopentasiloxane, and dodecamethylcyclohexasiloxane.

Examples of volatile hydrocarbon oils include paraffinic hydrocarbon oils such as n-decane, n-undecane, and n-dodecane; isoparaffinic hydrocarbon oils such as isodecane, isododecane, and hydrogenated polyisobutene; and cyclic paraffin hydrocarbon oils such as cyclodecane and cyclododecane. Of these, hydrocarbon oils having from 8 to 16 carbon atoms are preferred, hydrocarbon oils having from 10 to 16 carbon atoms are more preferred, and hydrocarbon oils having 12 carbon atoms are even more preferred.

As the volatile oil of the component (A2), one or more selected from the group consisting of isodecane, hexamethyldisiloxane, methyl trimethicone, and dimethylpolysiloxane having kinematic viscosity of 2 cSt or lower at 25°C are preferred, and one or more selected from the group consisting of isododecane, hexamethyldisiloxane, methyl trimethicone, and octamethyl dimethylpolysiloxane are more preferred. Of note, the kinematic viscosity can be measured by, for example, using an Ubbelohde's viscometer.

In view of increasing shrinkage and adhesiveness to the skin of the coating film, the component (A2) is more preferably one or more selected from the group consisting of isododecane, hexamethyldisiloxane, and dimethylpolysiloxane having kinematic viscosity of 1.5 cSt or lower at 25°C, even more preferably one or more selected from the group consisting of hexamethyldisiloxane and dimethylpolysiloxane having kinematic viscosity of 1 cSt or lower at 25°C, even more preferably hexamethyldisiloxane.

Examples of commercially available hexamethyldisiloxane and dimethylpolysiloxane having kinematic viscosity of 2 cSt or lower at 25°C include "KF-96L-0.65cs" (hexamethyldisiloxane), "TMF1.5," "KF-96L-1cs" (octamethyltrisiloxane), "KF-96L-1.5cs," and "KF-96L-2cs," which are manufactured by Shin-Etsu Chemical Co., Ltd., "SH200C Fluid 1cs" and "SH200C Fluid 1.5cs," which are manufactured by Dow Corning Toray Co., Ltd., "TSF451-0.65" manufactured by Momentive Performance Materials Japan LLC, and "BELSIL DM0.65" manufactured by Wacker Asahikasei Silicone Co., Ltd.

One or more different components (A2) can be used in combination. In view of increasing shrinkage and adhesiveness to the skin of the coating film, the content thereof is preferably 1 mass% or more, more preferably 30 mass% or more, even more preferably 50 mass% or more, even more preferably 70 mass% or more, and preferably 98 mass% or less, more preferably 97 mass% or less, even more preferably 94 mass% or less, even more preferably 93 mass% or less in the composition X. Further, the content of the component (A2) is preferably from 1 to 98 mass%, more preferably from 30 to 97 mass%, even more preferably from 50 to 94 mass%, even more preferably from 70 to 93 mass% in the composition X.

In the composition X, in view of increasing shrinkage and adhesiveness to the skin of the coating film, the mass ratio of the component (A1) to the component (A2), (A1)/(A2), is preferably 0.002 or higher, more preferably 0.02 or higher, even more preferably 0.04 or higher, even more preferably 0.05 or higher, and preferably 1 or lower, more preferably 0.7 or lower, even more preferably 0.4 or lower, even more preferably 0.2 or lower. Further, the mass ratio of the component (A1) to the component (A2), (A1)/(A2), is preferably from 0.002 to 1, more preferably from 0.02 to 0.7, even more preferably from 0.04 to 0.4. The mass ratio is even more preferably from 0.05 to 0.2.

<Component (A3)>

In the present invention, the composition X can further comprise a powder (A3), increases shrinkage and adhesiveness to the skin of the coating film, and when used in combination with the composition Y, can prevent an uncomfortable feeling.

Such a powder may be any of organic powders, inorganic powders, and the like.

Examples of organic powders include polyamide resins, nylon resins, polyester resins, polyethylene resins, polytetrafluoroethylene resins, polypropylene resins, polystyrene resins, benzoguanamine resins, polymethyl benzoguanamine resins, polymethyl methacrylate, polyurethane resins, vinyl resins, fluorine resins, acrylic resins, and melamine resins; silicone resins such as silicone elastomer obtained by crosslinking dimethylsilicone and polymethylsilsesquioxane; and crosslinked or uncrosslinked organic powders such as powders of one or more polymers or copolymers selected from the group consisting of poly(meth)acrylic acid, sodium poly(meth)acrylate, poly(meth)acrylic acid esters, and alkylene glycol poly(meth)acrylate such as butyl acrylate-vinyl acetate copolymers, styrene-acrylic acid copolymers, divinylbenzene-styrene copolymers, and (lauryl methacrylate/ethylene glycol dimethacrylate)copolymers.

Examples of inorganic powders include talc, kaolin, mica, sericite, phlogopite, magnesium aluminum silicate,

calcium silicate, aluminum silicate, magnesium silicate, barium silicate, strontium silicate, epoxy-treated aluminum, aluminum powder, calcium phosphate, silicic anhydride, anhydrous aluminum silicate, pyrophyllite clay, bentonite, smectite, montmorillonite, vermiculite, hectorite, zeolite, higilite, silica, alumina, zirconia, iron oxides (red iron oxide, yellow iron oxide, black iron oxide, and γ-iron oxide), yellow ochre, black titanium oxide, low-valent titanium oxide, iron titanate, zinc oxide, aluminum hydroxide, aluminum oxide, cobalt aluminum oxide, chromium oxide, zirconium oxide, titanium oxide, titanium oxide sol, iron oxide-titanium dioxide sinter, cerium oxide, magnesium oxide, chromium hydroxide, titanium-titanium dioxide sinter, cobalt titanate, manganese violet, cobalt violet, calcium carbonate, barium carbonate, magnesium carbonate, tungstate metal salts, barium sulfate, calcined calcium sulfate (calcined gypsum), bismuth oxychloride, calamine, sodium rosinate-treated magnesium oxide, fluorine apatite, hydroxyapatite, ceramic powder, and metal soap (such as zinc myristate, calcium palmitate, and aluminum stearate) and also include composite powders of two or more of these. Examples thereof further include organic-inorganic composite powders such as acrylic resin-coated aluminum powder and titanium oxide-coated nylon powder.

Examples of powders of natural fiber include silk powder, wool powder, and cellulose powder.

Examples of pearlescent pigments include titanated mica, iron oxide-coated titanated mica, titanium oxide-coated bismuth oxychloride, titanium oxide-coated talc, titanium oxide-coated glass flakes, and fish scale flakes.

These powders used as they are, or may be hydrophobized before use.

Such hydrophobization is not limited as long as it is a treatment applied to usual powders for cosmetics, and example thereof include a silicone treatment, an alkoxysilane treatment, a fatty acid treatment, a lauroyl lysine treatment, a lecitin treatment, a N-acyl amino acid treatment, a metal soap treatment, and a fluorine compound treatment. Of these, a silicone treatment is preferred. These treatments may be conducted by usual methods.

As the component (A3), one or more can be used in combination. In view of increasing shrinkage and adhesiveness to the skin of the coating film and preventing an uncomfortable feeling by use in combination with the composition Y, the content thereof is preferably 0.1 mass% or more, more preferably 1 mass% or more, even more preferably 2 mass% or more, even more preferably 3 mass% or more, and preferably 40 mass% or less, more preferably 20 mass% or less, even more preferably 15 mass% or less, even more preferably 10 mass% or less in the composition X. Further, the content of the component (A3) is preferably from 0.1 to 40 mass%, more preferably from 1 to 20 mass%, even more preferably from 2 to 15 mass%, even more preferably from 3 to 10 mass% in the composition X.

<Component (A4)>

In the present invention, the composition X can further comprise (A4) a surfactant, and preferred examples thereof include anionic surfactants, cationic surfactants, nonionic surfactants, and ampholytic surfactants.

Surfactants as the component (A4) are not limited as long as they are used for usual agents for skin external use, but they are preferably nonionic surfactants in view of being soluble in a volatile oil and preventing stickiness.

Examples of nonionic surfactants include sorbitan fatty acid ester, glycerin fatty acid ester, polyglycerin fatty acid ester, propylene glycol fatty acid ester, polyethylene glycol fatty acid ester, sucrose fatty acid ester, polyoxyethylene fatty acid ester, polyoxyethylene sorbitan fatty acid ester, polyoxyethylenealkyl ether, polyoxyethylene sorbitol fatty acid ester, polyoxyethylene glycerin fatty acid ester, polyoxyethylene propylene glycol fatty acid ester, polyoxyethylene castor oil, polyoxyethylene hydrogenated castor oil, polyoxyethylene hydrogenated castor oils fatty acid ester, polyoxyethylene phytostanol ether, polyoxyethylene phytosterose ether, polyoxyethylene cholestanol ether, polyoxyethylene cholesteryl ether, alkyl glyceryl ether-modified silicone, polyoxyalkylene-modified silicone, polyoxyalkylene-alkyl co-modified silicone, and polyoxyalkylene-fluoroalkyl co-modified silicone.

Of these, in view of increasing dispersibility/solubility of water and water-soluble components in an oil, the nonionic surfactant is preferably a silicone surfactant, and more preferably comprises one or more alkyl glyceryl ether-modified silicones and polyoxyalkylene-modified silicones, even more preferably comprises polyoxyalkylene-modified silicones.

Examples of polyoxyalkylene-modified silicones which can be used include commercially available products such as SH3771M, SH3772M, SH3773M, SH3775M, SH3749, and DC5200, which are manufactured by Dow Corning Toray Co., Ltd, and KF-6011, KF-6012, KF-6013, KF-6015, KF-6016, KF6017, and KF-6004, which are manufactured by Shin-Etsu Chemical Co., Ltd.

In view of increasing dispersibility/solubility of water and water-soluble components in an oil, the HLB value of a nonionic surfactant is preferably 1 or higher and 7 or lower, more preferably 2 or higher and 6 or lower.

Here, HLB (Hydrophilic-Lipophilic Balance) represents the proportion of the molecular weight of a hydrophilic portion to the molecular weight of the whole surfactant and can be obtained by the Griffin's formula for nonionic surfactants.

The HLB value of a mixed surfactant composed of two or more nonionic surfactants is obtained as follows. The HLB of the mixed surfactant is obtained by calculating an arithmetic mean of the HLB values of each nonionic surfactant based on the mixing ratio.

$$\text{Mixed HLB} = \Sigma(\text{HLBx} \times \text{Wx})/\Sigma\text{Wx}$$

HLBx represents the HLB value of a nonionic surfactant X.
Wx represents weight (g) of the nonionic surfactant X having the HLBx value.
One or more different nonionic surfactants can be used in combination, and in view of being soluble in a volatile oil and preventing stickiness, the content of the nonionic surfactants is preferably 30 mass% or less, more preferably 10 mass% or less, even more preferably 5 mass% or less, even more preferably 3 mass% or less, even more preferably 1 mass% or less in the composition X.

<Other components>

In the composition X, the content of water is preferably 50 mass% or less, more preferably 30 mass% or less, even more preferably 10 mass% or less, even more preferably 5 mass% or less, even more preferably 1 mass% or less in the composition X.
In addition to the above-described components, the composition X can optionally use various components usually used as long as the effect of the present invention is not impaired. Examples of these components include oily components other than described above, water-soluble polymers, antioxidants, ultraviolet absorbers, vitamins, preservatives, pH modifiers, flavors, plant extracts, moisturizers, colorants, cooling sensation agents, antiperspirants, sterilizers, and skin activators.
The composition X can be manufactured by usual methods using the components (A1) and (A2) and, if necessary, other components and can be applied to any forms such as aqueous compositions, oily compositions, and emulsified compositions. The composition can be applied as a preparation such as a liquid, a milky lotion, a paste, a cream, a gel, a solid, or a sheet.
The composition may be any of those which can be used for cosmetics, quasi-drugs, and drugs and can be used as skin care cosmetics such as lotions, milky lotions, creams, beauty essences, dispersions, gels, ointments, facial masks, mousses, aerosols, poultices, and cleansing agents.

<Component (B1)>

In the present invention, the component (B1) used in the composition Y is one or more selected from the group consisting of powders.
Powders as the component (B1) are not limited as long as they are used for usual cosmetics, and (B11) a colored pigment, extender pigment, or the like can be used.
Examples of (B11) colored pigments include inorganic pigments including metal oxides such as titanium oxide, cerium oxide, aluminum oxide, yellow iron oxide, black iron oxide, red iron oxide, prussian blue, ultramarine, chromium oxide, and chromium hydroxide, metal complexes such as manganese violet and cobalt titanate, and further carbon black; synthetic organic pigments such as Red No. 3, Red No. 104, Red No. 106, Red No. 201, Red No. 202, Red No. 204, Red No. 205, Red No. 220, Red No. 226, Red No. 227, Red No. 228, Red No. 230, Red No. 401, Red No. 405, Red No. 505, Orange No. 203, Orange No. 204, Orange No. 205, Yellow No. 4, Yellow No. 5, Yellow No. 401, Blue No. 1, and Blue No. 404; and natural organic dyes such as β-carotene, caramel, and paprika dye.
The average particle size of powders is preferably from 0.01 to 50 μm.
Of these, in view of preventing an uncomfortable feeling specific to polymers immediately after application of the composition X, increasing the adhesiveness of the film, and achieving coverage with the film appearing fine-textured in a natural state, the colored pigment (B11) preferably comprises one or more of titanium oxide, yellow iron oxide, black iron oxide, red iron oxide, more preferably comprises at least titanium oxide.
Examples of extender pigments include inorganic powders such as silicic acid, silicic anhydride, magnesium silicate, talc, sericite, mica, phlogopite, kaolin, clay, bentonite, bismuth oxychloride, zirconium oxide, zinc oxide, magnesium oxide, aluminum oxide, cerium oxide, calcium sulfate, barium sulfate, magnesium sulfate, calcium carbonate, magnesium carbonate, boron nitride, and alumina powder, and composite powders thereof.
Examples thereof also include polyamide resins, nylon resins, polyester resins, polyethylene resins, polytetrafluoroethylene resins, polypropylene resins, polystyrene resins, benzoguanamine resins, polymethyl benzoguanamine resins, polyurethane resins, vinyl resins, fluorine resins, acrylic resins, and melamine resins; and crosslinked or uncrosslinked organic powders such as powders of one or more polymers or copolymers selected from the group consisting of butyl acrylate-vinyl acetate copolymers, styrene-acrylic acid copolymers, silicone resins, and divinylbenzene-styrene copolymers, and composite powders thereof.
These powders used as they are, or may be hydrophobized before use.
Such hydrophobization is not limited as long as it is a treatment applied to usual powders for cosmetics, and

example thereof include a silicone treatment, a methylhydrogenpolysiloxane treatment, an alkoxysilane treatment, a fatty acid treatment, a lauroyl lysine treatment, a lecitin treatment, a N-acyl amino acid treatment, a metal soap treatment, and a fluorine compound treatment. Of these, a silicone treatment, a methylhydrogenpolysiloxane treatment, and an alkoxysilane treatment are preferred. These treatments may be conducted by usual methods.

As the component (B1), one or more can be used in combination. In view of preventing an uncomfortable feeling specific to polymers immediately after application of the composition X, increasing the adhesiveness of the film, and achieving coverage with the film appearing fine-textured in a natural state, the content thereof is preferably 0.1 mass% or more, more preferably 2 mass% or more, even more preferably 10 mass% or more, and preferably 99 mass% or less, more preferably 96 mass% or less, even more preferably 94 mass% or less in the composition Y. Further, the content of the component (B1) is preferably from 0.1 to 99 mass%, more preferably from 2 to 96 mass%, even more preferably from 10 to 94 mass% in the composition Y.

The component (B1) preferably comprises (B11) a colored pigment, and in view of preventing an uncomfortable feeling specific to polymers immediately after application of the composition X, increasing the adhesiveness of the film, and achieving coverage with the film appearing fine-textured in a natural state, the content of the colored pigment is preferably 0.1 mass% or more, more preferably 1 mass% or more, even more preferably 2 mass% or more, and preferably 40 mass% or less, more preferably 30 mass% or less, even more preferably 25 mass% or less in the composition Y. Further, the content of the colored pigment is preferably from 0.1 to 40 mass%, more preferably from 1 to 30 mass, even more preferably from 2 to 25 mass% in the composition Y.

In the composition Y, in view of preventing an uncomfortable feeling specific to polymers immediately after application of the composition X, increasing the adhesiveness of the film, and achieving coverage with the film appearing fine-textured in a natural state, the mass ratio of the component (B11) to the component (B1), (B11)/(B1) is preferably 0.1 or higher, more preferably 0.15 or higher, even more preferably 0.2 or higher and preferably 0.6 or lower, more preferably 0.5 or lower, even more preferably 0.45 or lower. Further, the mass ratio of the component (B11) to the component (B1), (B11)/(B1) is preferably from 0.1 to 0.6, more preferably from 0.15 to 0.5, even more preferably from 0.2 to 0.45.

<Other components>

In addition to the component (B1), the composition Y can optionally use various components usually used as long as the effect of the present invention is not impaired. Examples of these components include oily components, surfactants, water-soluble polymers, antioxidants, ultraviolet absorbers, vitamins, preservatives, pH modifiers, flavors, plant extracts, moisturizers, colorants, cooling sensation agents, antiperspirants, sterilizers, skin activators, and water.

The composition Y is a composition other than the composition X.

The composition Y can be manufactured by usual methods using the component (B1) and other components and can be applied to any forms such as aqueous compositions, oily compositions, and emulsified compositions. The composition can be applied as a preparation such as a liquid, a milky lotion, a paste, a cream, a gel, a solid, or a sheet.

The composition may be any of those which can be used for cosmetics, quasi-drugs, and drugs and can be used as skin care cosmetics such as lotions, milky lotions, creams, beauty essences, dispersions, gels, ointments, facial masks, spray types, mousses, aerosols, poultices, and cleansing agents; ultraviolet protection cosmetics such as sunscreen milky lotions and sunscreen creams; makeup cosmetics such as makeup bases, foundations, concealers, blushes, eye shadows, mascaras, eyeliners, eyebrows, overcoats, and lipsticks, and the like.

The makeup application method of the present invention comprises the steps of (A) applying to the skin the composition X and (B) applying to the skin the composition Y. The order of application of the composition X and composition Y is not limited, and it is sufficient that either of the steps comprises applying to the skin the composition X. For example, the composition Y may be applied after the composition X is applied, the composition X may be applied after the composition Y is applied, or further, the composition X and/or composition Y may be applied a plurality of times before or/and after either of the steps.

The application method is not particularly limited, and the composition may be either applied or, in the case of a liquid, sprayed or the like, to the skin.

The composition X and composition Y used in the present invention can be used to form a cosmetic kit comprising these composition X and composition Y, the kit being intended to be applied to the skin.

Regarding the above-described embodiments, the present invention further discloses the following methods and the like.

<1> A makeup application method characterized in comprising the steps of:

(A) applying to the skin a composition X comprising components (A1) and (A2):

(A1) a polymer comprising a norbornane structure and/or a silicone-modified Pullulan; and
(A2) a volatile oil; and

(B) applying to the skin a composition Y comprising a component (B1), other than the composition X:
(B1) one or more selected from the group consisting of powders.

<2> The makeup application method according to the above <1>, wherein the component (A1) is preferably (A11) a polymer comprising a norbornane structure, more preferably a norbornane structure-comprising silicone-modified polymer, even more preferably a polymer having a repeating unit of the following formula (1) or (2):

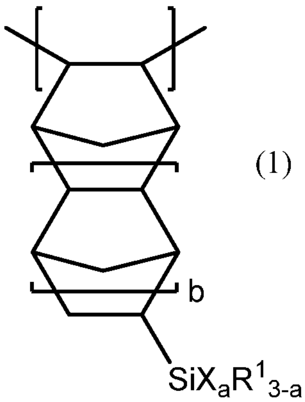

(1)

wherein $R^1$ is each independently an alkyl group having one or more and 12 or less carbon atoms, and X is a group of the following formula (i). a is an integer of 1 or above and 3 or below, and b is an integer of 0 or above and 2 or below.

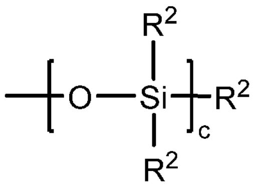

(i)

wherein $R^2$ is each independently a hydrocarbon group having one or more and 12 or less carbon atoms, and c is an integer of 1 or above and 5 or below.

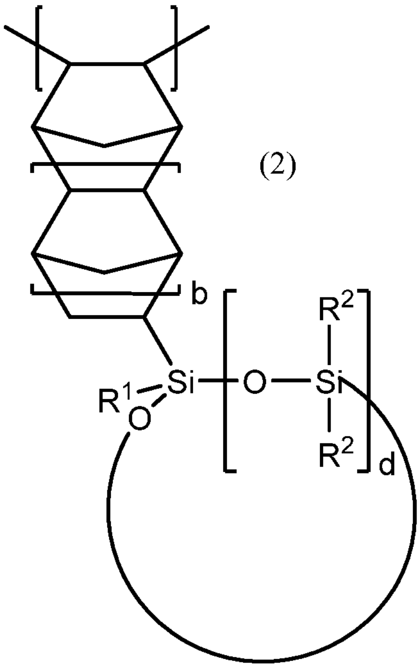

(2)

wherein $R^1$, $R^2$, and b are the same as described above, and d is an integer of 2 or above and 5 or below.

<3> The makeup application method according to the above <1> or <2>, wherein the component (A1) is preferably a silicone-modified polynorbornene of the following formula (6), more preferably a (norbornene/tris(trimethylsiloxy)silylnorbornene)copolyme r.

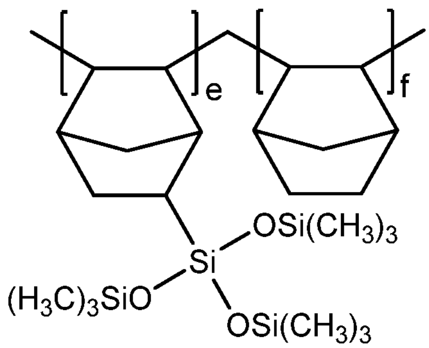

(6)

wherein e and f are the numbers of repeating units and each independently an integer of 1 or above.

<4> The makeup application method according to the above <3>, wherein, in the component (A), in the formula (6), the ratio of e and f, e/f, is preferably from 20/80 to 90/10 (mol/mol), more preferably from 30/70 to 80/20 (mol/mol), even more preferably from 50/50 to 70/30 (mol/mol) .

<5> The makeup application method according to the above <1>, wherein the component (A1) is preferably (A12) a silicone-modified Pullulan, more preferably a Pullulan having a silicone structure in a side chain, even more preferably a silicone-modified Pullulan in which at least some hydrogen atoms of an OH group in the Pullulan is substituted with a group of the following formula (7), even more preferably tri(trimethylsiloxy)silyl propyl carbamic acid Pullulan.

$$-Z^1\text{-}SiX_aR^1_{3-a} \qquad (7)$$

wherein $Z^1$ is a single bond or divalent organic group. $R^1$, X, and a are the same as described above, and in the same view, X is preferably trimethylsiloxy group, and a is preferably 3.

<6> The makeup application method according to any one of the above <1> to <5>, wherein the content of the solid content of the component (A1) is preferably 0.01 mass% or more, more preferably 1 mass% or more, even more preferably 2 mass% or more, even more preferably 4 mass% or more, even more preferably 5 mass% or more, and

preferably 30 mass% or less, more preferably 28 mass% or less, even more preferably 25 mass% or less, even more preferably 20 mass% or less, and even more preferably 18 mass% or less in the composition X.

<7> The makeup application method according to any one of the above <1> to <6>, wherein the component (A2) is preferably one or more selected from the group consisting of volatile silicone oils and volatile hydrocarbon oils, more preferably one or more selected from the group consisting of isododecane, hexamethyldisiloxane, methyl trimethicone, and dimethylpolysiloxane having kinematic viscosity of 2 cSt or lower at 25°C, even more preferably one or more selected from the group consisting of isododecane, hexamethyldisiloxane, methyl trimethicone, and octamethyl dimethylpolysiloxane.

<8> The makeup application method according to any one of the above <1> to <6>, wherein the component (A2) is preferably one or more selected from the group consisting of volatile silicone oils and volatile hydrocarbon oils, more preferably one or more selected from the group consisting of isododecane, hexamethyldisiloxane, and dimethylpolysiloxane having kinematic viscosity of 1.5 cSt or lower at 25°C, even more preferably one or more selected from the group consisting of hexamethyldisiloxane and dimethylpolysiloxane having kinematic viscosity of 1 cSt or lower at 25°C, even more preferably hexamethyldisiloxane.

<9> The makeup application method according to any one of the above <1> to <8>, wherein the content of the component (A2) is preferably 1 mass% or more, more preferably 30 mass% or more, even more preferably 50 mass% or more, even more preferably 70 mass% or more, and preferably 98 mass% or less, more preferably 97 mass% or less, even more preferably 94 mass% or less, even more preferably 93 mass% or less in the composition X.

<10> The makeup application method according to any one of the above <1> to <9>, wherein the mass ratio of the component (A1) to the component (A2), (A1)/(A2), is preferably 0.002 or higher, more preferably 0.02 or higher, even more preferably 0.04 or higher, even more preferably 0.05 or higher, and preferably 1 or lower, more preferably 0.7 or lower, even more preferably 0.4 or lower, even more preferably 0.2 or lower in the composition (X).

<11> A makeup application method comprising the steps of:

(A) applying to the skin a composition X comprising components (A1) and (A2):

(A1) from 0.01 to 30 mass% of a norbornane structure-comprising silicone-modified polymer, and
(A2) from 1 to 98 mass% of one or more volatile oil selected from the group consisting of volatile silicone oils and volatile hydrocarbon oils, and

(B) applying to the skin a composition Y comprising a component (B1), other than the composition X:
(B1) from 0.1 to 99 mass% of one or more selected from the group consisting of powders.

<12> The makeup application method according to any one of the above <1> to <11>, wherein the component (B1) preferably comprises (B11) a colored pigment, more preferably comprises one or more of titanium oxide, yellow iron oxide, black iron oxide, and red iron oxide, even more preferably comprises at least titanium oxide.

<13> The makeup application method according to any one of the above <1> to <12>, wherein the component (B1) preferably comprises (B11) a colored pigment, and the content of the colored pigment (B11) is preferably 0.1 mass% or more, more preferably 1 mass% or more, even more preferably 2 mass% or more, and preferably 40 mass% or less, more preferably 30 mass% or less, even more preferably 25 mass% or less in the composition Y.

<14> The makeup application method according to any one of the above <1> to <13>, wherein the component (B1) has been preferably hydrophobized, and the hydrophobization is more preferably a silicone treatment, a methylhydrogenpolysiloxane treatment, or an alkoxysilane treatment.

<15> The makeup application method according to any one of the above <1> to <14>, wherein the content of the component (B1) is preferably 0.1 mass% or more, more preferably 2 mass% or more, even more preferably 10 mass% or more, and preferably 99 mass% or less, more preferably 96 mass% or less, even more preferably 94 mass% or less in the composition Y.

<16> The makeup application method according to any one of the above <1> to <15>, wherein the mass ratio of the component (B11) to the component (B1), (B11)/(B1) is preferably 0.1 or higher, more preferably 0.15 or higher, even more preferably 0.2 or higher, and preferably 0.6 or lower, more preferably 0.5 or lower, even more preferably 0.45 or lower in the composition Y.

<17> The makeup application method according to any one of the above <1> to <16>, wherein the composition X further comprises preferably (A3) a powder, more preferably an inorganic powder.

<18> The makeup application method according to the above <17>, wherein the content of the component (A3) is preferably 0.1 mass% or more, more preferably 1 mass% or more, even more preferably 2 mass% or more, even more preferably 3 mass% or more, and preferably 40 mass% or less, more preferably 20 mass% or less, more preferably 15 mass% or less, even more preferably 10 mass% or less in the composition X.

<19> The makeup application method according to any one of the above <1> to <18>, wherein composition X further

can comprise (A4) a surfactant, preferably a nonionic surfactant, even more preferably a nonionic surfactant having HLB of from 1 to 7.
<20> The makeup application method according to the above <19>, wherein the content of the nonionic surfactant is preferably 30 mass% or less, more preferably 10 mass% or less, even more preferably 5 mass% or less, even more preferably 3 mass% or less, even more preferably 1 mass% or less in the composition X.
<21> The makeup application method according to any one of the above <1> to <20>, wherein in the composition X, the content of water is preferably 50 mass% or less, more preferably 30 mass% or less, even more preferably 10 mass% or less, even more preferably 5 mass% or less, even more preferably 1 mass% or less in the composition X.
<22> The makeup application method according to any one of the above <1> to <21>, wherein the composition Y is applied after the composition X is applied.
<23> The makeup application method according to any one of the above <1> to <21>, wherein the composition X is applied after the composition Y is applied.
<24> A cosmetic kit to be applied to the skin, comprising:

(A) a composition X comprising components (A1) and (A2):

(A1) a polymer comprising a norbornane structure and/or a silicone-modified Pullulan; and
(A2) a volatile oil; and

(B) a composition Y comprising a component (B1), other than the composition X:
(B1) one or more selected from the group consisting of powders.

<25> A cosmetic kit to be applied to the skin, comprising:

(A) a composition X comprising components (A1) and (A2):

(A1) from 0.01 to 30 mass% of a norbornane structure-comprising silicone-modified polymer; and
(A2) from 1 to 98 mass% of one or more volatile oils selected from the group consisting of volatile silicone oils and volatile hydrocarbon oils; and

(B) a composition Y comprising a component (B1), other than the composition X:
(B1) from 0.1 to 99 mass% of one or more selected from the group consisting of powders.

## Examples

The present invention is described below with reference to examples, but the scope of the present invention is not limited to these examples. Of note, various measurements and assessments were performed by the following methods in the examples.

(Adhesiveness of film)

Five expert panelists applied each composition to the skin, and then, sensory evaluation was performed with the following criteria to assess adhesiveness between the coating film and the skin. The result was expressed as the sum of scores given by the five panelists.

5; The skin and the coating film are attached to each other, and no twists occur.
4; The skin and the coating film are attached to each other, and not much twists occur.
3; The skin and the coating film are attached to each other, and twists slightly occur.
2; The coating film floats from the skin, and twists occur.
1; The coating film floats from the skin, and twists markedly occur.

(Absence of uncomfortable feeling (creakiness) immediately after application)

Five expert panelists applied each composition to the skin, and then, sensory evaluation was performed with the following criteria to assess an uncomfortable feeling of the skin. The result was expressed as the sum of scores given by the five panelists.

5; No uncomfortable feeling (creakiness) of the skin is felt at all.

4; No uncomfortable feeling of the skin is felt.
3; An uncomfortable feeling of the skin is not much felt.
2; An uncomfortable feeling of the skin is slightly felt.
1; An uncomfortable feeling of the skin is felt.

(Appearance of film (fine texture))

Five expert panelists applied each composition to the skin, and then, sensory evaluation was performed with the following criteria to assess finish of the coating film. The result was expressed as the sum of scores given by the five panelists.

5; Looks considerably fine-textured.
4; Looks fine-textured.
3; Looks slightly fine-textured.
2; Does not look much fine-textured.
1; Does not look fine-textured.

(Appearance of film (natural))

Five expert panelists applied each composition to the skin, and then, sensory evaluation was performed with the following criteria to assess natural finish of the coating film. The result was expressed as the sum of scores given by the five panelists.

5; Looks considerably natural.
4; Looks natural.
3; Looks slightly natural.
2; Does not look much natural.
1; Does not look natural.

Examples 1 to 7, Comparative Examples 1 to 3

Compositions X having the compositions shown in Table 1 and makeup cosmetics Y having the compositions shown in Tables 2 to 4 were manufactured, and the compositions each were applied to the skin by the steps shown in Table 5.

At this time, adhesiveness of the film, absence of an uncomfortable feeling (creakiness) immediately after application, appearance of the film (fine texture), and state of the film (natural) were assessed. The results are shown in Table 3.

(Manufacturing method)

(1) Composition X:

An oil phase component obtained by mixing the components (A1) and (A2) and other components were dispersed with a disperser and stirred with a homo mixer to obtain each of the compositions X.

(2) Composition Y;

(2-1) Makeup cosmetic Y1 (powder foundation) solid powder cosmetic;

All the powder components comprising the component (B1) were mixed and pulverized, and then the other oil solution was added thereto and mixed. The resulting mixture was further pulverized with a pulverizer. The pulverized product was filled into a container and molded at a molding pressure of from 1,000 to 2,500 kgf to thereby obtain a solid powder cosmetic.

(2-2) Makeup cosmetic Y2 (cosmetic base) water-in-oil emulsified cosmetic;

All the powder components comprising the component (B1) were mixed and pulverized, the pulverized product was added to other oil phase components separately mixed, and the mixture was dispersed with a disperser. Thereafter,

a water phase component was added and dispersed with a disperser and stirred with a homo mixer to obtain a water-in-oil emulsified cosmetic.

(2-3) Makeup cosmetic Y3 (liquid foundation) water-in-oil emulsified cosmetic;

All the powder components comprising the component (B1) were mixed and pulverized, the pulverized product was added to other oil phase components separately mixed, and the mixture was dispersed with a disperser. Thereafter, a water phase component was added and dispersed with a disperser and stirred with a homo mixer to obtain water-in-oil emulsified cosmetics.

(Application method)

In Examples 1 to 4, Examples 6 and 7, and Comparative Example 3, an appropriate amount of the composition X of each of the examples was applied with fingers to the area from the lips to the entire cheek in a half of the face of a subject, and then, an appropriate amount of the composition Y was applied with a sponge puff thereto.

In Example 5 and Comparative Example 2, an appropriate amount of the composition Y of each of the examples was applied with a sponge puff to the area from the lips to the entire cheek in a half of the face of a subject, and then, an appropriate amount of the composition X was applied with fingers thereto.

In Comparative Example 1, an appropriate amount of the composition X was applied with fingers to the area from the lips to the entire cheek in a half of the face of a subject.

[Table 1]

| Component (mass%) | | Raw material name | Composition X | | | | | |
|---|---|---|---|---|---|---|---|---|
| | | | 1 | 2 | 3 | 4 | 5 | 6 |
| Component (A1) | (Norbornene/tris (trimethylsiloxy) silylnorbornene copolymer solid content *1 | NBN-30-ID manufactured by Shin-Etsu Chemical Co., Ltd. | 13.0 | 13.0 | 2.0 | 18.0 | | |
| | Silicone-modified Pullulan solid content *2 | TSPL-30-ID manufactured by Shin-Etsu Chemical Co., Ltd. | | | | | 13.0 | |
| Other | Acrylic silicone solid content *3 | KP-550 manufactured by Shin-Etsu Chemical Co., Ltd. | | | | | | 13.0 |
| Component (A2) | Hexamethyldisiloxane | KF-96L-0.65cs manufactured by Shin-Etsu Chemical Co., Ltd. | 56.7 | 51.5 | 77.1 | 34.8 | 51.5 | 51.5 |
| | Isododecane | Marukasol R manufactured by Maruzen Petrochemical Co., Ltd. | 30.3 | 30.3 | 15.7 | 42.0 | 30.3 | 30.3 |

(continued)

| Component (mass%) | | Raw material name | Composition X | | | | | |
|---|---|---|---|---|---|---|---|---|
| | | | 1 | 2 | 3 | 4 | 5 | 6 |
| Component (A3) | Dimethicone-treated silica | SA-SB-300 (refractive index: 1.54; average particle size: 3-7 μm) manufactured by Miyoshi Kasei, Inc. | | 5.0 | 5.0 | 5.0 | 5.0 | 5.0 |
| Other | Poluglyceryl diisostearate-2 | COSMOL 42V manufactured by The Nisshin OilliO Group, Ltd. | | 0.2 | 0.2 | 0.2 | 0.2 | 0.2 |
| Total | | | 100.0 | 100.0 | 100.0 | 100.0 | 100.0 | 100.0 |
| (A1) | | | 13.0 | 13.0 | 2.0 | 18.0 | 13.0 | 0.0 |
| (A2) | | | 87.0 | 81.8 | 92.8 | 76.8 | 81.8 | 81.8 |
| (A3) | | | 0.0 | 5.0 | 5.0 | 5.0 | 5.0 | 5.0 |

*1 to *3 in Table 1 are as follows:
*1: "NBN-30-ID" (an isododecane solution of a norbornene/tris(trimethylsiloxy)silylnorbornene copolymer with e/f of 60/40 (mol/mol) in the following formula (6) and Mn of 360,000; effective concentration: 30 mass%) manufactured by Shin-Etsu Chemical Co., Ltd.

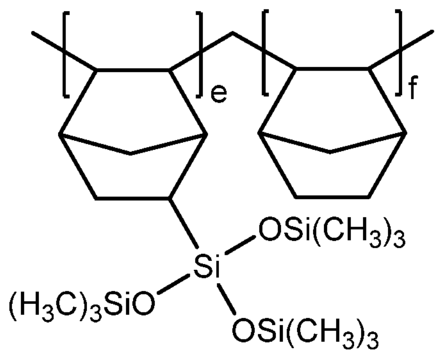

(6)

*2: "TSPL-30-ID" (an isododecane solution of tri(trimethylsiloxy)silyl propyl carbamide acid Pullulan; effective concentration: 30 mass%) manufactured by Shin-Etsu Chemical Co., Ltd. was dried under reduced pressure at 50°C for 12 hours, and the obtained solid content was used.

*3: "KP-550" (an isododecane solution of a graft copolymer comprising an acrylic polymer and dimethylpolysiloxane, effective concentration: 40 mass%) manufactured by Shin-Etsu Chemical Co., Ltd. was dried under reduced pressure at 50°C for 12 hours, and the obtained solid content was used.

[Table 2]

| | **Makeup cosmetic Y1 (powder foundation) solid powder cosmetic** | |
|---|---|---|
| | (Component) | (mass%) |
| (B11) | Silica-coated titanium oxide (i-NAFLECS IWS22S13 manufactured by Nippon Sheet Glass Co., Ltd.) | 5 |

(continued)

| | Makeup cosmetic Y1 (powder foundation) solid powder cosmetic | |
|---|---|---|
| | (Component) | (mass%) |
| (B11) | Dimethylpolysiloxane-treated titanium oxide (CR-50 manufactured by Ishihara Sangyo Kaisha, Ltd.) | 10 |
| (B11) | Methylhydrogenpolysiloxane-treated titanium oxide (MT-600B manufactured by TAYCA CORPORATION) | 3 |
| (B11) | Methylhydrogenpolysiloxane-treated yellow iron oxide | 2.1 |
| (B11) | Methylhydrogenpolysiloxane-treated red iron oxide | 0.5 |
| (B11) | Methylhydrogenpolysiloxane-treated black iron oxide | 0.4 |
| (B1) | (Vinyl dimethicone/methicone silsesquioxane) crosspolymer (KSP-100 manufactured by Shin-Etsu Chemical Co., Ltd.) | 2.5 |
| (B1) | (Diphenyl dimethicone/vinyl diphenyl dimethicone/silsesquioxane) crosspolymer (KSP-300 manufactured by Shin-Etsu Chemical Co., Ltd.) | 2.5 |
| (B1) | Polymethylsilsesquioxane | 3.5 |
| (B1) | Dimethylpolysiloxane-treated boron nitride (SHP-3 manufactured by Mizushima Ferroalloy Co., Ltd.) | 22 |
| (B1) | Dimethylpolysiloxane-treated talc | 27.5 |
| (B1) | Methylhydrogenpolysiloxane-treated synthetic phlogopite (PDM-NFE manufactured bv Topy Industries, Ltd.) | 10 |
| (B1) | Methylhydrogenpolysiloxane-treated zinc oxide | 4 |
| | 2-Ethylhexyl p-methoxycinnamate | 4 |
| | Vegetable squalane | 3 |
| | Total | 100 |
| | (B1) Powder | 93 |
| | (B1 1) Colored pigment | 21 |
| | (B11)/(B1) | 0.226 |

[Table 3]

| | Makeup cosmetic Y2 (cosmetic base) water-in-oil emulsified cosmetic | |
|---|---|---|
| | (Component) | (mass%) |
| (B11) | Silicone-treated microparticulate titanium dioxide (MT-500SA manufactured by TAYCA CORPORATION) | 1 |
| (B11) | Silicone-treated microparticulate titanium dioxide (STR-100W manufactured by Sakai Chemical Industry Co., Ltd.) | 4 |
| (B11) | Silicone-treated red iron oxide | 0.01 |
| (B11) | Silicone-treated yellow iron oxide | 0.03 |
| (B11) | Silicone-treated black iron oxide | 0.001 |
| (B11) | Titanium oxide (CR-50 manufactured by Ishihara Sangyo Kaisha, Ltd.) | 0.56 |
| (B1) | Silicone-treated microparticulate zinc oxide (MZY-505M manufactured by TAYCA CORPORATION) | 9 |
| (B1) | Silicone-coated talc | 2 |

(continued)

| | Makeup cosmetic Y2 (cosmetic base) water-in-oil emulsified cosmetic | |
|---|---|---|
| | (Component) | (mass%) |
| | Dimethylpolysiloxane (KF-96A-1000CS manufactured by Shin-Etsu Chemical Co., Ltd.) | 5 |
| | Trimethylsiloxysilicic acid (BELSIL TMS 803 SILICONE RESIN manufactured by Wacker Asahikasei Silicone Co., Ltd.) solid content | 3 |
| | Dimethylpolysiloxane (KF-96A-1CS manufactured by Shin-Etsu Chemical Co., Ltd.) | 4.9 |
| | Dimethylpolysiloxane (KF-96A-2CS manufactured by Shin-Etsu Chemical Co., Ltd.) | 26.5 |
| | Octyl p-methoxycinnamate | 4 |
| | Polyether-modified silicone (SH3375M manufactured by Dow Corning Toray Co., Ltd.) | 2.5 |
| | Ethanol | 6.5 |
| | Purified water | 30.999 |
| | Total | 100 |
| | (B1) Powder | 16.601 |
| | (B1 1) Colored pigment | 5.601 |
| | (B11)/(B1) | 0.337 |

[Table 4]

| | Makeup cosmetic Y3 (liquid foundation) water-in-oil emulsified cosmetic | |
|---|---|---|
| | (Component) | (mass%) |
| (B11) | 2 mass% Caprylyltriethoxysilane-treated red iron oxide | 0.4 |
| (B11) | 2 mass% Caprylyltriethoxysilane-treated black iron oxide | 0.2 |
| (B11) | 2 mass% Caprylyltriethoxysilane-treated yellow iron oxide | 1.6 |
| (B11) | 2 mass% Caprylyltriethoxysilane-treated titanium oxide (CR-50 manufactured by Ishihara Sangyo Kaisha, Ltd.)) | 8 |
| (B1) | Silicone-treated microparticulate zinc oxide (MZY-505M manufactured by TAYCA CORPORATION) | 6 |
| (B1) | Spherical silsesquioxane resin (Tospearl 145A) | 2 |
| (B1) | Silicone-coated talc | 6.2 |
| | Dimethylpolysiloxane (KF-96A-100CS manufactured by Shin-Etsu Chemical Co., Ltd.) | 1 |
| | Acrylic silicone dendrimer (FA40001CM manufactured by Dow Corning Toray Co., Ltd.) solid content | 3 |
| | Dimethylpolysiloxane (KF-96A-1CS manufactured by Shin-Etsu Chemical Co., Ltd.) | 5.5 |
| | Dimethylpolysiloxane (KF-96A-2CS manufactured by Shin-Etsu Chemical Co., Ltd.) | 22 |
| | Cyclopentasiloxane | 11 |
| | Octyl p-methoxycinnamate | 3 |

(continued)

| | Makeup cosmetic Y3 (liquid foundation) water-in-oil emulsified cosmetic | |
|---|---|---|
| | (Component) | (mass%) |
| | Polyether-modified silicone (SH3375M manufactured by Dow Corning Toray Co., Ltd.) | 0.6 |
| | Ethanol | 7 |
| | Glycerin | 4 |
| | Purified water | 18.5 |
| | Total | 100 |
| | (B1) Powder | 24.4 |
| | (B11) Colored pigment | 10.2 |
| | (B1 1)/(B1) | 0.418 |

[Table 5]

| | | Example 1 | Example 2 | Example 3 | Example 4 | Example 5 | Example 6 | Example 7 | Comparative Example 1 | Comparative Example 2 | Comparative Example 3 |
|---|---|---|---|---|---|---|---|---|---|---|---|
| Step 1 | | Composition X-1 | Composition X-2 | Composition X-3 | Composition X-4 | Makeup cosmetic Y1 | Composition X-2 | Composition X-5 | Composition X-2 | Makeup cosmetic Y1 | Composition X-6 |
| Step 2 | | Makeup cosmetic Y1 | Makeup cosmetic Y2 | Makeup cosmetic Y3 | Makeup cosmetic Y3 | Composition X-2 | Makeup cosmetic Y1 | Makeup cosmetic Y1 | - | Composition X-6 | Makeup cosmetic Y1 |
| Evaluation | Adhesiveness to the skin | 25 | 21 | 23 | 25 | 21 | 21 | 17 | 9 | 7 | 8 |
| | Absence of uncomfortable feeling immediately after application | 22 | 22 | 25 | 20 | 25 | 25 | 18 | 10 | 7 | 8 |
| | Appearance of film (fine texture) | 25 | 23 | 21 | 24 | 21 | 21 | 15 | 10 | 8 | 7 |
| | State of film (natural) | 21 | 25 | 25 | 21 | 21 | 20 | 17 | 9 | 9 | 9 |

## Claims

1. A makeup application method comprising the steps of:

   (A) applying to the skin a composition X comprising components (A1) and (A2):

   (A1) a polymer comprising a norbornane structure and/or a silicone-modified Pullulan; and
   (A2) a volatile oil; and

   (B) applying to the skin a composition Y comprising a component (B1), other than the composition X:
   (B1) one or more selected from the group consisting of powders.

2. The makeup application method according to Claim 1, wherein the component (A1) is a norbornane structure-comprising silicone-modified polymer.

3. The makeup application method according to Claim 1 or 2, wherein the component (A1) is a silicone-modified polynorbornene of the following formula (6):

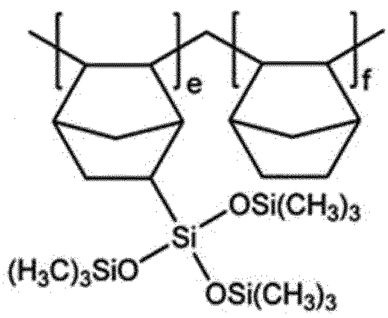
(6)

wherein e and f are the numbers of repeating units and are each independently an integer of 1 or above.

4. The makeup application method according to any one of Claims 1 to 3, wherein the content of the component (A1) in the composition X is from 0.01 to 30 mass%.

5. The makeup application method according to any one of Claims 1 to 4, wherein the component (A2) in the composition X is one or more selected from the group consisting of volatile silicone oils and volatile hydrocarbon oils.

6. The makeup application method according to any one of Claims 1 to 5, wherein the content of the component (A2) in the composition X is from 1 to 98 mass%.

7. The makeup application method according to any one of Claims 1 to 6, wherein the content of the component (B1) in the composition Y is from 0.1 to 99 mass%.

8. A cosmetic kit to be applied to the skin, comprising:

   (A) a composition X comprising components (A1) and (A2):

   (A1) a polymer comprising a norbornane structure and/or a silicone-modified Pullulan; and
   (A2) a volatile oil; and

   (B) a composition Y comprising a component (B1), other than the composition X:
   (B1) one or more selected from the group consisting of powders.

## INTERNATIONAL SEARCH REPORT

International application No.
PCT/JP2021/011782

A. CLASSIFICATION OF SUBJECT MATTER
A61Q 1/00(2006.01)i; A61Q 1/02(2006.01)i; A61Q 1/12(2006.01)i; A61K 8/29(2006.01)i; A61K 8/73(2006.01)i; A61K 8/81(2006.01)i; A61K 8/89(2006.01)i; A61K 8/92(2006.01)i
FI: A61K8/81; A61K8/73; A61K8/92; A61K8/29; A61K8/89; A61Q1/00; A61Q1/02; A61Q1/12

According to International Patent Classification (IPC) or to both national classification and IPC

B. FIELDS SEARCHED

Minimum documentation searched (classification system followed by classification symbols)
A61Q1/00; A61Q1/02; A61Q1/12; A61K8/29; A61K8/73; A61K8/81; A61K8/89; A61K8/92

Documentation searched other than minimum documentation to the extent that such documents are included in the fields searched

| | |
|---|---|
| Published examined utility model applications of Japan | 1922–1996 |
| Published unexamined utility model applications of Japan | 1971–2021 |
| Registered utility model specifications of Japan | 1996–2021 |
| Published registered utility model applications of Japan | 1994–2021 |

Electronic data base consulted during the international search (name of data base and, where practicable, search terms used)
Mintel GNPD

C. DOCUMENTS CONSIDERED TO BE RELEVANT

| Category* | Citation of document, with indication, where appropriate, of the relevant passages | Relevant to claim No. |
|---|---|---|
| Y | JP 2012-17317 A (SHIN-ETSU CHEMICAL CO., LTD.) 26 January 2012 (2012-01-26) claims 1, 4, paragraphs [0088], [0092] | 1-8 |
| Y | JP 2001-278729 A (SHISEIDO CO., LTD.) 10 October 2001 (2001-10-10) claims 1, 5, example 27 | 1-8 |
| Y | JP 10-45536 A (KANEBO KABUSHIKI KAISHA) 17 February 1998 (1998-02-17) claims 1, 3, examples | 1-8 |
| A | JP 2011-26263 A (SHIN-ETSU CHEMICAL CO., LTD.) 10 February 2011 (2011-02-10) entire text | 1-8 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>23 April 2021 (23.04.2021) | Date of mailing of the international search report<br>18 May 2021 (18.05.2021) |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

International application No.
PCT/JP2021/011782

C (Continuation). DOCUMENTS CONSIDERED TO BE RELEVANT

| Category* | Citation of document, with indication, where appropriate, of the relevant passages | Relevant to claim No. |
|---|---|---|
| A | JP 2002-322043 A (POLA CHEMICAL INDUSTRIES, INC.) 08 November 2002 (2002-11-08) entire text | 1-8 |
| A | JP 2010-174099 A (SHIN-ETSU CHEMICAL CO., LTD.) 12 August 2010 (2010-08-12) entire text | 1-8 |

Form PCT/ISA/210 (continuation of second sheet) (January 2015)

**INTERNATIONAL SEARCH REPORT**
Information on patent family members

International application No.
PCT/JP2021/011782

| Patent Documents referred in the Report | Publication Date | Patent Family | Publication Date |
|---|---|---|---|
| JP 2012-17317 A | 26 Jan. 2012 | US 2011/0301247 A1<br>claims 1, 4,<br>paragraphs [0100],<br>[0102]-[0105]<br>EP 2444062 A2<br>CN 102274133 A<br>KR 10-2011-0134301 A | |
| JP 2001-278729 A | 10 Oct. 2001 | (Family: none) | |
| JP 10-45536 A | 17 Feb. 1998 | (Family: none) | |
| JP 2011-26263 A | 10 Feb. 2011 | US 2011/0028571 A1<br>EP 2347794 A2<br>CN 101984953 A | |
| JP 2002-322043 A | 08 Nov. 2002 | (Family: none) | |
| JP 2010-174099 A | 12 Aug. 2010 | US 2010/0190950 A1<br>EP 2213691 A1<br>KR 10-2010-0087643 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2012017317 A


### Non-patent literature cited in the description


- International Cosmetic Ingredient Dictionary and Handbook. 2016, vol. 2, 2274